# EUROPEAN PATENT APPLICATION

(11) **EP 2 572 704 A1**
(43) Date of publication of application: **27.03.2013**
(21) Application number: 12185407.9
(22) Date of filing: 21.09.2012
(51) Int. Cl.: A61K 9/00, A61K 9/20, A61K 9/50, A61K 31/40, A61K 9/28, A61P 5/50

(54) **Orally-Disintegrating Formulations of Vildagliptin**

(30) Priority: 22.09.2011 TR 201109315
(71) Applicant: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Saydam, Mehtap, 34460 Istanbul (TR); Yelken, Gülay, 34460 Istanbul (TR)
(74) Representative: Sevinç, Erkan

(57) **Abstract**

The present invention relates to an orally-disintegrating pharmaceutical tablet formulation, which is characterized by comprising vildagliptin or a pharmaceutically acceptable salt of vildagliptin and a coating which is selected from methyl methacrylate - diethyl aminoethyl methacrylate copolymer, polyvinylpyrrolidone having 3.5 - 5.5 mPa s or 5.5 - 8.5 mPa s viscosity, hydroxypropyl methyl cellulose having 3.5 - 5.5 mPa s viscosity.

## Description

### Field of Invention

The present invention relates to a formulation comprising vildagliptin or a pharmaceutically acceptable salt of vildagliptin. The present invention particularly relates to an orally-disintegrating tablet formulation of vildagliptin, which is stable against ambient influences.

### Background of Invention

Vildagliptin is a dipeptidyl dipeptidase-IV (DPP-IV) inhibitor developed for use in the treatment of type 2 diabetes (non-insulin dependent diabetes). Vildagliptin inhibits the degradation of the dipeptidyl dipeptidase-IV enzyme, thereby inhibiting the effects of incretin hormones, glucagon-like peptide-1 (GLP-1), and of glucose-dependent insulinotropic peptide (GIP). The chemical designation of vildagliptin is (S)-{[(3-hydroxyadamantan-1-yl)amino]acetyl}pyrrolidine-2-carbonitril, with the chemical structure illustrated below in Formula 1.

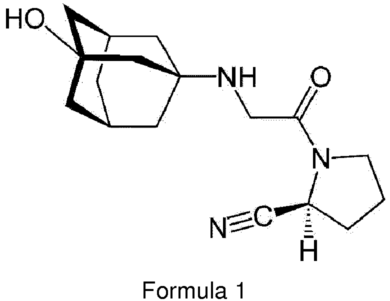

Vildagliptin is soluble in water and in organic polar solvents.

Vildagliptin is marketed under the trademark Galvus^{®} in 50 mg dosage forms. It is used against diabetes mellitus, but particularly in treating type 2 diabetes.

There are various patents in the patent literature in relation to vildagliptin.

The patent application WO0034241 discloses vildagliptin or an acid addition salt thereof, as well as its use in diabetes mellitus and obesity.

The patent application WO2006078593 claims a direct-compression formulation of a DPP-IV inhibitor compound, and preferably of vildagliptin or an acid addition salt thereof.

The patent application WO2006135723 discloses a formulation, comprising vildagliptin as an active agent, as well as hydroxypropyl methyl cellulose, microcrystalline cellulose, and magnesium stearate.

Many conventional tablet formulations of vildagliptin are disclosed in the patents referred to above. This active pharmaceutical agent used for treating many diseases, however, is preferred in an orally-disintegrating form. These preparations are advantageous for use in younger, elder, and noncompliant patients. Orally-disintegrating dosage forms are convenient when drinking water is not available or preferable.

On the other hand, various difficulties are encountered in formulating the orally-disintegrating dosage forms. Developing orally-disintegrating formulations is difficult due to several factors. First of all, the time required for the disintegration of the dosage form in the oral cavity under the presence of saliva is shorter than the time required by the stomach. Therefore, these formulations are quite porous and therefore are not too hard. Such porous formulations are prone to be susceptible to humidity. As a result of this, some stability problems may be encountered. In addition to these, measures must be taken while preparing, packaging, handling, and storing finished dosage forms of orally-disintegrating formulations.

For this reason, orally-disintegrating compositions of vildagliptin or a pharmaceutically acceptable salt of vildagliptin is desired, which would overcome the drawbacks referred to hereinabove and would let the active agent be disintegrated and dissolved in the oral cavity.

In addition to the stability problems possibly to arise from the porous structure of the orally-disintegrating tablet form of vildagliptin, other stability problems are frequently encountered in vildagliptin formulations under the influence of ambiance and physical conditions. Vildagliptin is an active agent that is highly-susceptible to air and humidity conditions. When vildagliptin is initially exposed to air and humidity, it degrades structurally and develops chemical behavioral changes. As a result of this, two main problems emerge. The first problem is that the stability of the products developed is not at a desired level and the shelf life thereof is shortened. Secondly, vildagliptin is reactive against the excipients employed in developing formulations containing the same. This fact causes impurities and unwanted components to be included into the formulation.

In result, due to the abovementioned drawbacks, a novelty is required in the art of vildagliptin formulations, used for preventing and treating diabetes.

### Object and Brief Description of Invention

The present invention provides an easily-administrable vildagliptin formulation, eliminating all problems referred to above and bringing additional advantages to the relevant prior art.

Accordingly, the main object of the present invention is to obtain an orally-disintegrating novel formulation having antidiabetic activity.

A further object of the present invention is to obtain a stable vildagliptin formulation having high bioavailability.

A further object of the present invention is to provide a high disintegration rate for said formulation.

A further object of the present invention is to provide a high dissolution rate and solubility for such stable vildagliptin formulations having high bioavailability.

A further object of the present invention is to avoid any aggregation of ingredients of the composition and to provide a desired level of flowability during production, thanks to convenient excipients used while the formulation is obtained.

An orally-disintegrating pharmaceutical tablet formulation has been developed to carry out any objects, referred to above and to emerge from the following detailed description.

In a preferred embodiment according to the present invention, said novelty is carried out with vildagliptin or a pharmaceutically acceptable salt of vildagliptin and and a coating which is selected from methyl methacrylate - diethyl aminoethyl methacrylate copolymer, polyvinylpyrrolidone having 3.5 - 5.5 mPa s or 5.5 - 8.5 mPa s viscosity, hydroxypropyl methyl cellulose having 3.5 - 5.5 mPa s viscosity.

The formulation disintegrates in the oral cavity in 90 seconds and preferably in 40 seconds.

In a preferred embodiment according to the present invention, vildagliptin-containing granules are coated with a coating which is selected from methyl methacrylate - diethyl aminoethyl methacrylate copolymer, polyvinylpyrrolidone having 3.5 - 5.5 mPa s or 5.5 - 8.5 mPa s viscosity, hydroxypropyl methyl cellulose having 3.5 - 5.5 mPa s viscosity.

In another preferred embodiment according to the present invention, the amount said coating layer is 0.01 to 20% by weight, preferably 0.1 to 10% by weight, and more preferably 0.2 to 4% by weight of the total amount of granules.

In another preferred embodiment of the present invention, the tablet hardness is 5 to 100 N, preferably 10 to 60 N, and more preferably 15 to 40 N.

Another preferred embodiment of the present invention comprises at least one or more excipient(s).

In another preferred embodiment according to the present invention, one or more pharmaceutically acceptable excipient(s) is/are selected from the group consisting of disintegrants, diluents, binders, lubricants, glidants, sweeteners, flavoring agents, preserving agents and coloring agents.

In another preferred embodiment according to the present invention, said disintegrants are selected from the group consisting of at least one or a mixture of croscarmellose sodium, crospovidone, low-substituted hydroxypropyl cellulose, sodium starch glycolate, and soy polysaccharides (Emcosoy^{®}).

In another preferred embodiment according to the present invention, the weight proportion of vildagliptin to the disintegrant is between 1:10 and 10:1.

In another preferred embodiment according to the present invention, said disintegrant is preferably croscarmellose sodium.

In another preferred embodiment according to the present invention, said diluent is preferably mannitol and/or microcrystalline cellulose.

In another preferred embodiment according to the present invention, said binder is preferably polyvinylpyrrolidone and/or hydroxypropyl methyl cellulose.

In another preferred embodiment according to the present invention, said lubricant is preferably magnesium stearate and sodium stearyl fumarate.

In another preferred embodiment according to the present invention, said glidant is preferably colloidal silicon dioxide.

In another preferred embodiment according to the present invention, said sweetener is preferably aspartam, sucralose and/or saccharine.

In another preferred embodiment according to the present invention, said flavoring agent is preferably menthol and/or fruit extracts.

In another preferred embodiment according to the present invention, said pharmaceutical formulation is consisted of
(a) vildagliptin or a pharmaceutically acceptable salt of vildagliptin at approximately 5 to 60% by weight,
(b) polyvinyl alcohol or methacrylic acid polymer at 0.25 to 20% by weight,
(c) croscarmellose sodium at approximately 0.1 to 30% by weight,
(d) mannitol at approximately 1 to 60% by weight,
(e) microcrystalline cellulose at approximately 1 to 30% by weight,
(f) polyvinylpyrrolidone at approximately 1 to 20% by weight,
(g) colloidal silicon dioxide at approximately 0.01 to 5% by weight,
(h) magnesium stearate and sodium lauryl sulfate at approximately 0.1 to 5% by weight,
(i) sucralose at approximately 0.01 to 5% by weight,
(j) menthol and/or fruit extracts at approximately 0.01 to 5% by weight.

In a further preferred embodiment according to the present invention, said formulation is in the form of a tablet.

Another preferred embodiment according to the present invention provides a method for preparing said pharmaceutical formulation, this method comprising the steps of
(a) coating vildagliptin or a pharmaceutically acceptable salt of vildagliptin with a coating agent which is dissolved in a suitable solvent,
(b) mixing the coated granules obtained above with other excipients,
(c) blending this mixture with a lubricant and a glidant, and
(d) compressing the blended mixture into tablets.

### Detailed Description of Invention

The present invention is described with the following example in more details. This example is not limiting the scope of the present invention and must be considered under the light of the foregoing detailed disclosure. It is obvious that those skilled in the relevant art can produce similar embodiments under the light of the foregoing disclosures by making many adaptations on both the materials and methods, without departing from the scope of the present invention.

This orally-disintegrating tablet composition composed of the followings is designed to minimize the disintegration time of tablets and maximize the mechanical resistance.

### Example 1

An orally-disintegrating tablet composition is composed of the following ingredients:
(a) vildagliptin or a pharmaceutically acceptable salt of vildagliptin at approximately 5 to 60% by weight,
(b) methyl methacrylate - diethyl aminoethyl methacrylate copolymer at 0.25 to 20% by weight,
(c) croscarmellose sodium at approximately 0.1 to 30% by weight,
(d) mannitol at approximately 1 to 60% by weight,
(e) microcrystalline cellulose at approximately 1 to 30% by weight,
(f) polyvinylpyrrolidone at approximately 1 to 20% by weight,
(g) colloidal silicon dioxide at approximately 0.01 to 5% by weight,
(h) magnesium stearate and sodium lauryl sulfate at approximately 0.1 to 5% by weight,
(i) sucralose at approximately 0.01 to 5% by weight,
(j) menthol and/or orange extract at approximately 0.01 to 5% by weight.

### Example 2

An orally-disintegrating tablet composition is composed of the following ingredients:
(a) vildagliptin or a pharmaceutically acceptable salt of vildagliptin at approximately 5 to 60% by weight,
(b) polyvinylpyrrolidone having 3.5 - 5.5 mPa s or 5.5 - 8.5 mPa s viscosity at 0.25 to 20% by weight,
(c) croscarmellose sodium at approximately 0.1 to 30% by weight,
(d) mannitol at approximately 1 to 60% by weight,
(e) microcrystalline cellulose at approximately 1 to 30% by weight,
(f) polyvinylpyrrolidone at approximately 1 to 20% by weight,
(g) colloidal silicon dioxide at approximately 0.01 to 5% by weight,
(h) magnesium stearate and sodium lauryl sulfate at approximately 0.1 to 5% by weight,
(i) sucralose at approximately 0.01 to 5% by weight,
(j) menthol and/or orange extract at approximately 0.01 to 5% by weight.

### Example 3

An orally-disintegrating tablet composition is composed of the following ingredients:
(a) vildagliptin or a pharmaceutically acceptable salt of vildagliptin at approximately 5 to 60% by weight,
(b) hydroxypropyl methyl cellulose having 3.5 - 5.5 mPa s viscosity.at 0.25 to 20% by weight,
(c) croscarmellose sodium at approximately 0.1 to 30% by weight,
(d) mannitol at approximately 1 to 60% by weight,
(e) microcrystalline cellulose at approximately 1 to 30% by weight,
(f) polyvinylpyrrolidone at approximately 1 to 20% by weight,
(g) colloidal silicon dioxide at approximately 0.01 to 5% by weight,
(h) magnesium stearate and sodium lauryl sulfate at approximately 0.1 to 5% by weight,
(i) sucralose at approximately 0.01 to 5% by weight,
(j) menthol and/or orange extract at approximately 0.01 to 5% by weight.

The production process is conducted preferably by admixing the ingredients, blending this admixture with lubricant(s) and glidant(s), and compressing the blended mixture into tablets.

The orally-disintegrating formulations according to the present invention can be prepared by techniques such as direct compression, dry granulation, wet granulation, etc. well known to those skilled in the art. Orally-disintegrating compositions according to the present invention can also be prepared by means of technologies such as spray drying, freeze drying, floss formation process, die casting, Zydis^{®} technology, Flashtab technology, OraSolv^{®} technology, DuraSolv^{®} technology, Wowtab^{™} (nonaqueous immediately-soluble tablet) technology and other similar technologies. Vildagliptin or a pharmaceutically acceptable salt thereof and other excipients are preferably mixed together and then the resulting mixture is compressed to give tablets.

Coated vildagliptin active agent is used in the formulation. Accordingly, a coating process is conducted that efficiently prevents the contact of such granules with air and humidity in order to provide stability for the granules of vildagliptin or a pharmaceutically acceptable salt of vildagliptin. The process steps are as follows. Methyl methacrylate - diethyl aminoethyl methacrylate copolymer or polyvinylpyrrolidone having 3.5 - 5.5 mPa s or 5.5 - 8.5 mPa s viscosity or hydroxypropyl methyl cellulose having 3.5 - 5.5 mPa s viscosity are dissolved in a suitable solvent. The solution obtained is spray-coated onto the granules or powders comprising vildagliptin or a pharmaceutically acceptable salt of vildagliptin to perform the coating operation. Then the coated granules are dried. This coating layer may optionally be a single layer or a multilayer. Coated vildagliptin granules can be used in the content of medicaments which are efficient in preventing or treating the diabetes disease. Orally-disintegrating tablets can be compressed by making use of coated vildagliptin granules and convenient excipients.

The orally-disintegrating pharmaceutical formulation obtained has surprisingly shown fairly-good dissolution rates and stability. The formulation disintegrates in the oral cavity in 90 seconds and preferably in 40 seconds. The tablet hardness according to this invention is 5 to 100 N, preferably 10 to 60 N, and more preferably 15 to 40 N. Disintegration is achieved in a desired time interval by applying a compressive force in said ranges and adding proper disintegrants. The amount of methyl methacrylate - diethyl aminoethyl methacrylate copolymer or polyvinylpyrrolidone having 3.5 - 5.5 mPa s or 5.5 - 8.5 mPa s viscosity or hydroxypropyl methyl cellulose having 3.5 - 5.5 mPa s viscosity in said coating layer is 0.01 to 20% by weight, preferably 0.1 to 10% by weight, and more preferably 0.2 to 4% by weight of the total amount of granules. Coated granule or granules bring the stability of the formulation to a desired level. Thanks to the coating developed, the solubility of the active agent is not influenced in the absorption region. This also avoids the aggregation of granules and any reduction in their flowability during production.

In another process, in turn, vildagliptin and a binder are dissolved or dispersed in a suitable solvent. Sugar/microcrystalline spheres are coated with this solution and the granules obtained are coated with methyl methacrylate - diethyl aminoethyl methacrylate copolymer, polyvinylpyrrolidone having 3.5 - 5.5 mPa s or 5.5 - 8.5 mPa sviscosity, hydroxypropyl methyl cellulose having 3.5 - 5.5 mPa s viscosity solution to complete the coating process. The coated granules (pellets) are mixed with the other excipients in the formulation, then a lubricant is added to the mixture, mixed for a short time, and then the mixture is compressed into tablets.

In a further process, in turn, vildagliptin and a binder are dissolved or dispersed in methyl methacrylate - diethyl aminoethyl methacrylate copolymer or polyvinylpyrrolidone or hydroxypropyl methyl cellulose and then are subjected to spray-drying. The granules obtained are mixed with the other excipients in the formulation, then a lubricant is added to the mixture, mixed shortly, and then the mixture is compressed into tablets.

In another process, in turn, vildagliptin and methyl methacrylate - diethyl aminoethyl methacrylate copolymer or polyvinylpyrrolidone or hydroxypropyl methyl cellulose are dissolved with a suitable solvent. While this solution is mixed at a determined speed and temperature, a solvent is introduced so that the polymer and the active agent are precipitated. If necessary, the precipitation can be assisted by using pH regulating agents. While the polymers are precipitated they coat the active agent so as to give spherical micro particles during this process. The coated particles are dried and sieved to provide a proper particle size distribution. Coated particles are mixed with the other excipients in the formulation, then a lubricant is added to the mixture, mixed shortly, and then the mixture is compressed into tablets.

In another process, in turn, it is melted with a meltable polymer and passed through an extruder, then subjected to a spheronization step and sieved to give a proper particle size distribution. The particles obtained are mixed with the other excipients in the formulation, then a lubricant is added to the mixture, mixed shortly, and then the mixture is compressed into tablets.

The present invention is used for treating the diabetes disease.

Orally-disintegrating formulations according to the present invention comprises tablets, single-dose packages, mini tablets, and multilayered and multicoated tablets, pellets, or powders formulated according to the standard methods of the relevant art. The formulation is preferably in the form of tablets.

The orally-disintegrating tablet composition according to the present invention is preferably in the form of a disk, circle, sphere, donut, bar, polygon, ellipse, etc..

The term granule, as used and defined herein, means powder, particle, granule, or pellet forms of vildagliptin or of a pharmaceutically acceptable salt of vildagliptin.

Pharmaceutical formulations according to the present invention may also comprise one or more pharmaceutically acceptable excipient(s). Such pharmaceutically acceptable excipients include, but are not limited to disintegrants, fillers, glidants, lubricants, coating agents, surface active agents, etc. and the mixtures thereof.

Suitable disintegrants include, but are not limited to alginic acid and alginates, ion-exchange resins, magnesium aluminum silicate, sodium dodecyl sulfate, sodium carboxymethyl cellulose, croscarmellose sodium, cross-linked PVP, carboxymethyl cellulose calcium, docusate sodium, guar gum, low-substituted HPC, polacrilin potassium, poloxamer, povidone, sodium alginate, sodium glycine carbonate and sodium lauryl sulfate, etc., and the mixtures thereof.

Suitable fillers include sugars, mannitol, sorbitol, sucrose, inorganic salts, calcium salts, polysaccharides, dextrose, dicalcium phosphate, sodium chloride, dextrates, lactitol, maltodextrin, sucrose-maltodextrin mixtures, xylitol, trehalose, heavy magnesium carbonate, galen IQ (isomalt), LudiFlash (mannitol, crospovidone and polyvinyl acetate), Pharmaburst, Panexcea (microcrystalline cellulose, HPMC and crospovidone), F-Melt.

Suitable binders include, but are not limited to polyvinylpyrrolidone, polyethylene glycol, polyvinyl alcohol, hydroxypropyl methyl cellulose, carboxymethyl cellulose, methyl cellulose and other cellulose derivatives, starch, collagen, gelatin, sugars, aluminum hydroxide, polyvinyl alcohol, carrageenan, bentonite, laponite, and similar inorganic agents; cetostearyl alcohol, polyoxyethylene-alkyl ethers, and other surfactants, starch mucilage, acacia mucilage, polydextrose, polyethylene oxide, pullulan, guar gum, xanthan gum and similar agents, and the mixtures thereof.

Suitable lubricants include, but are not limited to magnesium stearate, calcium stearate, sodium stearyl fumarate, sodium lauryl sulfate, silica, talk, calcium stearate, polyethylene glycol, paraffin, sodium stearyl fumarate, sodium lauryl sulfate, magnesium lauryl sulfate, fumaric acid, glyceryl palmitostearate, hydrogenated vegetable oils, zinc stearate, stearic acid, etc., and the mixtures thereof. Even if the preferred lubricants are magnesium stearat and sodium stearyl fumarate, other less hydrophobic lubricants may be employed in certain circumstances not to adversely influence the hydrophobicity, i.e. a combination of magnesium stearate and sodium lauryl sulfate may be used for this purpose.

Suitable glidants include, but are not limited to colloidal silicon dioxide, talk, aluminum silicate, etc., and the mixtures thereof.

Suitable coating agents include, but are not limited to poloxamer, polyacrylamide, hydroxypropyl cellulose, ethyl cellulose, hydroxypropyl methyl cellulose, methyl cellulose, and similar semi-synthetic polymers, polymethacrylates (e.g. Eudragit E and RD types), polyvinyl acetate, cellulose acetate phthalate, ethylene vinyl acetate, methyl aminoethyl methacrylate, neutral methacrylic acid esters, polyactide, polyactide co-glycolide, low molecular weight polyethylene/polyisobutylene, polyanhydrides and poly(ortho-ester)s, diethyl aminoethyl methacrylate, Kollicoat smartseal 30D etc., and the mixtures thereof.
The orally-disintegrating compositions according to the present invention can also include sweeteners and flavoring agents to enhance patient compliance.

Suitable sweeteners include, but are not limited to aspartam, acesulfame-K, sucralose, saccharine; glucose, lactose, fructose and other sugars, and mannitol, sorbitol, xylitol, erythritol, and other sugar alcohols, etc., and the mixtures thereof.

Suitable flavoring agents include, but are not limited to menthol, peppermint, cinnamon, chocolate, vanilin, and fruit extracts such as cherry, orange, banana, berry, lemon, cardamom, anise, strawberry, grape, and the mixtures thereof.

Suitable colorants include, but are not limited to at least one or a mixture of food, drug, and cosmetic (FD&C) dyes (e.g. FD&C blue, FD&C green, FD&C red, FD&C yellow, FD&C lake), ponceau, indigo drug & cosmetic (D&C) blue, indigotine FD&C blue, carmoisine indigotine (indigo Carmine); iron oxides (e.g. iron oxide red, yellow, black), quinoline yellow, flame red, brilliant red (carmine), carmoisine, sunset yellow, etc., and the mixtures thereof.

## Claims

1. An orally-disintegrating pharmaceutical tablet formulation, **characterized by** comprising vildagliptin or a pharmaceutically acceptable salt of vildagliptin and a coating which is selected from methyl methacrylate - diethyl aminoethyl methacrylate copolymer, polyvinylpyrrolidone having 3.5 - 5.5 mPa s or 5.5 - 8.5 mPa sviscosity, hydroxypropyl methyl cellulose having 3.5 - 5.5 mPa s viscosity.

2. The pharmaceutical formulation according to Claim 1, wherein vildagliptin-containing granules are coated with a coating which is selected from methyl methacrylate - diethyl aminoethyl methacrylate copolymer, polyvinylpyrrolidone having 3.5 - 5.5 mPa s or 5.5 - 8.5 mPa sviscosity, hydroxypropyl methyl cellulose having 3.5 - 5.5 mPa s viscosity.

3. The pharmaceutical formulation according to any of the preceding claims, **characterized in that** the tablet hardness is 5 to 100 N, preferably 10 to 60 N, and more preferably 15 to 40 N.

4. The pharmaceutical formulation according to any of the preceding claims, wherein the amount of said coating layer is 0.01 to 20% by weight, preferably 0.1 to 10% by weight, and more preferably 0.2 to 4% by weight of the total granule amount.

5. The pharmaceutical formulation according to any of the preceding claims, further comprising at least one or more than one excipient.

6. The pharmaceutical formulation according to any of the preceding claims, wherein said one or more pharmaceutically acceptable excipient(s) is/are selected from the group consisting of disintegrants, diluents, binders, glidants, lubricants, sweeteners, flavoring agents, and coloring agents.

7. The pharmaceutical formulation according to any of the preceding claims, wherein said disintegrants are selected from the group consisting of at least one or a mixture of croscarmellose sodium, crospovidone, low-substituted hydroxypropyl cellulose, sodium starch glycolate, and soy polysaccharides.

8. The pharmaceutical formulation according to any of the preceding claims, wherein the weight ratio of vildagliptin to the disintegrant is in the range of 1:10 to 10:1.

9. The pharmaceutical formulation according to any of the preceding claims, wherein the disintegrant is preferably croscarmellose sodium.

10. The pharmaceutical formulation according to any of the preceding claims, wherein said diluent is preferably mannitol and/or microcrystalline cellulose.

11. The pharmaceutical formulation according to any of the preceding claims, wherein said binder is preferably polyvinylpyrrolidone and/or hydroxypropyl methyl cellulose.

12. The pharmaceutical formulation according to any of the preceding claims, wherein said lubricant is preferably magnesium stearate and sodium stearyl fumarate.

13. The pharmaceutical formulation according to any of the preceding claims, wherein said glidant is preferably colloidal silicon dioxide.

14. The pharmaceutical formulation according to any of the preceding claims, wherein said sweetener is preferably aspartam, sucralose and/or saccharine.

15. The pharmaceutical formulation according to any of the preceding claims, wherein said flavoring agent is preferably menthol and/or fruit extracts.

16. A method for preparing a pharmaceutical formulation according to any of the preceding claims, comprising the steps of
(a) coating vildagliptin or a pharmaceutically acceptable salt of vildagliptin with a coating agent which is dissolved in a suitable solvent,
(b) mixing the coated granules obtained above with other excipients,
(c) blending this mixture with a lubricant and a glidant, and
(d) compressing the blended mixture into tablets.

17. The pharmaceutical formulation according to any of the preceding claims for use in preventing or treating the diabetes disease in mammalians and particularly in humans.
